# EUROPEAN PATENT APPLICATION

(11) **EP 4 432 301 A1**
(43) Date of publication of application: **18.09.2024**
(21) Application number: 22907881.1
(22) Date of filing: 13.12.2022
(51) Int. Cl.: G16H 50/70, G16H 50/50, G16H 50/20, G16B 40/20, G16H 30/20, G16H 30/40, G16H 70/60, G16B 45/00, G06T 7/00, G06T 7/11

(54) **METHOD FOR TRAINING ARTIFICIAL NEURAL NETWORK PROVIDING DETERMINATION RESULT OF PATHOLOGY SPECIMEN, AND COMPUTING SYSTEM PERFORMING SAME**

(30) Priority: 13.12.2021 KR 20210177378
(71) Applicant: Deep Bio Inc., Seoul 08380 (KR)
(72) Inventor: KWAK, Tae Yeong, Seoul 05119 (KR); CHANG, Hye Yoon, Seoul 03303 (KR); CHO, Joon Young, Seoul 08394 (KR); KIM, Sun Woo, Seongnam-si, Gyeonggi-do 13599 (KR)
(74) Representative: Flügel Preissner Schober Seidel
(86) International application number: PCT/KR2022/020180
(87) International publication number: WO 2023/113414

(57) **Abstract**

Disclosed are: a method for extracting only a portion stained with a specific dye from a pathology slide stained with a mixed dye in which various types of dyes are mixed, training an artificial neural network, and determining a pathology image stained by using various staining techniques; and a computing system performing same. According to an aspect of the present invention, provided is an invention in which a neural network learning system generates and learns a learning data set including M pieces of individual learning data (where M is a natural number greater than or equal to 2).

## Description

### Technical Field

The present disclosure relates to a method of training an artificial neural network which provides a determination result of a pathology specimen and a computing system performing the same. More specifically, the present disclosure relates to a method which is capable of perform determination on stained pathology images through various staining techniques and a computing system performing the same by extracting only parts stained with a specific staining agent from a pathology slide stained with a mixture of various types of stains and training an artificial neural network.

### Background Art

As the average life expectancy of modern people increases, the incidence of serious diseases such as cancer is also increasing, and pathology diagnosis methods such as checking expression of specific biomarkers and analyzing the state of tumor microenvironment (TME) are becoming more advanced in order to predict the prognosis of patients diagnosed with cancer and determine treatment methods.

Hematoxylin-eosin staining (hereinafter, referred to as H&E staining) is a method of staining the cell nucleus and stroma area with hematoxylin and eosin in purple and pink, respectively, and is the most basic pathology diagnosis method used by pathologists to identify morphological characteristics of cells and tissues of specimens through light microscopy.

Immunohistochemistry (IHC) staining is a method which is widely used to measure an expression level of a biomarker such as a specific protein in tissues, and a target biomarker present in tissues based on antigen-antibody binding can be stained with brown diaminobenzidine (DAB) dye to determine its expression level, and hematoxyline staining is used as a counterstain.

Cocktail staining or multiplex IHC staining methods, in which multiple biomarkers are stained simultaneously to measure expression levels and analyze positional relationships among them, are also in the practical application stage, where staining of various colors such as red or blue in addition to brown is used, and hematoxylin staining is still used as a counterstain.

In most cases, it is desirable to measure the expression of these biomarkers limited to cancer lesions. For example, in the case of a Ki-67 index, which indicates the degree of proliferation of cancer cells by utilizing a Ki-67 protein temporarily expressed during cell division, it is appropriate to measure, targeting only cancer cells. Since normal cells may also express Ki-67 during cell division, normal cell areas need to be excluded when measuring the expression level of these biomarkers. When analyzing the positional relationships among biomarkers, it is also meaningful to analyze the positional relationship centered on cancer cells, and therefore, distinguishing between cancer lesions and normal cell areas is a very important task in pathological diagnosis.

Meanwhile, as image analysis deep learning technology develops, attempts are being made to apply and commercialize deep learning technology in various tasks in the medical field. In the field of pathology diagnosis, instead of the existing method of making a diagnosis by a pathologist speculating and reading stained tissue slides at high magnification through an optical microscope, a method of converting tissue slides into high-resolution digital images through a digital slide scanner and then having a pathologist read the images through a computer monitor is becoming increasingly practical, and furthermore, products are emerging that allow pathologists to make faster and more accurate diagnoses by referring to results of image analysis through deep learning technology.

A method of diagnosing cancer and analyzing its severity by applying image analysis deep learning technology to a whole-slide image (WSI), which is an image generated by scanning the entire slide, has already reached a commercial level, and there are products approved as medical devices in Korea and abroad for some types of cancer, such as prostate cancer. All of these products perform functions such as analyzing WSIs generated from H&E-stained slides to determine the presence or absence of cancerous lesions, or to visualize location of detected lesions.

In an advanced automated pathology diagnosis system, such as automatically measuring the level of expression of a biomarker, it is necessary to detect and measure the expression of the biomarker by analyzing WSI generated from various types of staining slides other than H&E staining. Here, it is important to detect and locate a cancer lesion in the image, so it is necessary to develop automated methods to perform these functions.

When developing a method to detect the lesion by analyzing H&E-stained WSI using image analysis deep learning technology, a pathologist with expertise in the cancer lesion reviews the H&E stained WSI used in the development of deep learning technology and performs area annotation for the lesion. This data building process is equally necessary for the development of methods for detecting lesions by analyzing different types of stained WSIs. In other words, in order to develop a method for detecting lesions from WSI of a target stain other than H&E staining, a pathologist must review stained WSI and annotate the lesion area.

Since WSIs generated from various IHC-stained or cocktail-stained slides may have different color distribution patterns according to the staining method, there is a concern that detection performance will decrease significantly when deep learning technology for detecting cancer lesions generated from a specific stained WSI is applied to a different type of stained WSI. On the other hand, targets to be stained through the IHC staining method may be newly created as disease research progresses, and therefore, it is not realistic to utilize all existing IHC-stained and cocktail-stained images for technology development. In other words, there is a problem in that it is difficult to develop a lesion detection method required for an advanced automatic pathology diagnosis system with the current deep learning technology application method, which requires a pathologist to annotate the lesion area and build data for each stained WSI.

### Disclosure of the Invention

### Technical Goals

A technical object to be achieved by the present disclosure is a universal method and system that is capable of detecting lesions caused by diseases such as cancer by analyzing pathology images generated from pathology slides produced by staining with various staining methods.

In addition, another object is to provide a universal lesion detection method and system to detect lesions caused by cancer by analyzing pathology images generated from pathology slides produced by staining specific targets with DAB and other colors and staining cell nuclei with hematoxylin, regardless of the type of target.

### Technical Solutions

According to one aspect of the present disclosure, there is provided a method of training an artificial neural network including generating, by a neural network training system, a training dataset including M pieces of individual training data (where M is a natural number greater than or equal to 2), and training, by the neural network training system, the artificial neural network by inputting each of the M pieces of individual training data included in the training dataset to the artificial neural network, wherein the generating of the training dataset including the M pieces of individual training data includes generating m-th training data to be included in the training dataset for all m where 1<=m<=M, and the generating of the m-th training data includes acquiring an m-th original pathology image, where the m-th original pathology image is a pathology image stained with a mixed stain in which a predetermined stain to be extracted and one or more stains excluding the stain to be extracted are mixed, performing color deconvolution for extracting a part stained by the stain to be extracted from the m-th original pathology image to generate an m-th pathology image for training corresponding to the m-th original pathology image, and generating the m-th pathology image for training based on the m-th original pathology image.

In one embodiment, the stain to be extracted may be a hematoxylin stain.

In one embodiment, a part of the first to M-th original pathology images and another part of the first to M-th original pathology images may be pathology images stained with different mixed stains.

In one embodiment, the extracting of the part stained by the stain to be extracted from the m-th original pathology image to generate the m-th pathology image for training corresponding to the m-th original pathology image may include converting a signal intensity for each channel in a color space representing the m-th original pathology image into optical density, and converting the optical density into staining intensity according to a predetermined correlation formula.

According to another aspect of the present disclosure, there is provided a method of providing a determination result for a predetermined determination target pathology specimen through an artificial neural network trained by the artificial neural network training method including acquiring, by a computing system, a determination target pathology image stained with a mixed stain in which the stain to be extracted and one or more stains excluding the stain to be extracted are mixed, generating, by the computing system, an extraction image corresponding to the the determination target pathology image by extracting a part stained by the stain to be extracted from the determination target pathology image, and outputting, by the computing system, a determination result for the determination target pathology specimen by the artificial neural network based on the extraction image.

In one embodiment, at least a part of the determination target image and a part of the first to M-th original pathology images may be pathology images stained with different mixed stains.

In one embodiment, a lesion area caused by a predetermined disease may be annotated in the m-th original pathology image, and the generating of the m-th training data based on the m-th original pathology image may include generating the m-th training data by annotating the lesion area annotated in the m-th original pathology image to the m-th pathology image for training.

According to another aspect of the present disclosure, there is provided a computer program which is installed in a data processing device and recorded on a non-transitory medium for performing the method as described above.

According to another aspect of the present disclosure, there is provided a non-transitory computer-readable recording medium on which a computer program for performing the method as described above is recorded.

According to another aspect of the present disclosure, there is provided an artificial neural network training system including a processor and a memory in which a computer program is stored,wherein the computer program is configured to, when executed by the processor, cause the computing system to perform a method of training an artificial neural network, and the method of training the artificial neural network includes generating, by the neural network training system, a training dataset including M pieces of individual training data (where M is a natural number greater than or equal to 2), and training, by the neural network training system, the artificial neural network by inputting each of the M pieces of individual training data included in the training dataset to an input layer of the artificial neural network, wherein the generating of the training dataset including the M pieces of individual training data includes generating m-th training data to be included in the training dataset for all m where 1<=m<=M, and the generating of the m-th training data includes acquiring an m-th original pathology image, where the m-th original pathology image is a pathology image stained with a mixed stain in which a predetermined stain to be extracted and one or more stains excluding the stain to be extracted are mixed and a lesion area caused by a predetermined disease is annotated in the m-th original pathology image, performing color deconvolution for extracting a part stained by the stain to be extracted from the m-th original pathology image to generate an m-th pathology image for training corresponding to the m-th original pathology image, and generating the m-th training data by annotating the lesion area annotated in the m-th original pathology image to the m-th pathology image for training.

In one embodiment, the stain to be extracted may be a hematoxylin stain.

In one embodiment, a part of the first to M-th original pathology images and another part of the first to M-th original pathology images may be pathology images stained with different mixed stains.

In one embodiment, the extracting of the part stained by the stain to be extracted from the m-th original pathology image to generate the m-th pathology image for training corresponding to the m-th original pathology image may include converting a signal intensity for each channel in a color space representing the m-th original pathology image into optical density, and converting the optical density into staining intensity according to a predetermined correlation formula.

According to another aspect of the present disclosure, there is provided a determination result providing system for a pathology specimen including a processor, and a memory in which a computer program is stored, wherein the computer program is configured to, when executed by the processor, cause the determination result providing system to perform a method of providing a determination result for the pathology specimen through an artificial neural network trained by the method according to claim 1, and the method of providing the determination result includes acquiring, by the determination result providing system, a determination target pathology image stained with a mixed stain in which the stain to be extracted and one or more stains excluding the stain to be extracted are mixed, generating, by the determination result providing system, an extraction image corresponding to the the determination target pathology image by extracting a part stained by the stain to be extracted from the determination target pathology image, andoutputting, by the determination result providing system, a determination result for the determination target pathology specimen by the artificial neural network based on the extraction image.

In one embodiment, at least a part of the determination target image and a part of the first to M-th original pathology images may be pathology images stained with different mixed stains.

### Advantageous Effects

According to the technical idea of the present disclosure, there is an effect of effectively training an artificial neural network which is able to make determination about a lesion or disease using images stained by different methods. In other words, even if multiple images used as training data are not pathology images stained by the same method, there is an effect that they can be used as training data for training an artificial neural network by the neural network training method.

In addition, in the case of conventional technology for determination on pathology images using an artificial neural network, training and determination are made on pathology images stained in a specific method, but according to the technical idea of the present disclosure, there is an effect of enabling training and determination through images stained with different staining methods. For example, there is an effect of being able to detect cancer lesions by analyzing pathology images generated from slides produced by staining a specific target with DAB or another color and staining cell nuclei with hematoxylin, regardless of the type of target.

### Brief Description of Drawings

In order to more fully understand the drawings cited in the detailed description of the present disclosure, a brief description of each drawing is provided.
FIG. 1 is a schematic diagram illustrating an environment in which an artificial neural network training method and a determination result providing method for a pathology specimen are performed according to the technical idea of the present disclosure.
FIG. 2 is a flowchart for illustrating a neural network training method according to one embodiment of the present disclosure.
FIG. 3A is a diagram showing an example of an original pathology image, and FIG. 3B is a diagram showing a lesion area annotated to the pathology image of FIG. 3A.
FIG. 3C is an example of an image extracted from the original pathology image shown in FIG. 3A of a part stained by a stain to be extracted.
FIG. 3D shows training data generated from the original pathology image tagged with annotation information as shown in FIG. 3B.
FIG. 4 is an example illustrating a process of generating an image for training corresponding to the original pathology image according to one embodiment of the present disclosure.
FIG. 5 is a flowchart illustrating an example of a determination result providing method for a pathology specimen according to one embodiment of the present disclosure.
FIG. 6 is a diagram illustrating a schematic configuration of an artificial neural network training system according to one embodiment of the present disclosure, and FIG. 7 is a diagram illustrating a schematic configuration of a determination result providing system according to one embodiment of the present disclosure.

### Best Mode for Carrying Out the Invention

Since the present disclosure may be modified variously and have various embodiments, specific embodiments will be illustrated in the drawings and described in detail in the detailed description. However, this is not intended to limit the present disclosure to specific embodiments, and it should be understood to include all modifications, equivalents, and substitutes included in the spirit and scope of the present disclosure. In describing the present invention, if it is determined that a detailed description of related known technologies may obscure the gist of the present disclosure, the detailed description will be omitted.

Terms such as first and second may be used to describe various components, but the components should not be limited by the terms. Terms such as first and second do not indicate a particular order and are used only for the purpose of distinguishing one component from another component.

The terms used in the present application are used only to describe a particular embodiment and are not intended to limit the present disclosure. Singular expressions include plural expressions unless the context clearly means otherwise.

In this specification, it should be understood that terms such as "comprises", "include" or "have" are intended to designate the presence of features, numbers, steps, operations, components, parts, or combinations thereof described in the specification, but do not preclude the possibility of the presence or addition of one or more other features, numbers, steps, operations, components, parts, or combinations thereof.

Additionally, in this specification, when one component 'transmits' data to another component, this means that the component may transmit the data directly to the other component or transmit the data to the other component through at least one other component. Conversely, when one component 'directly transmits' data to another component, it means that the data is transmitted from the component to the other component without going through still other component.

Hereinafter, with reference to the accompanying drawings, the present disclosure will be described in detail focusing on embodiments of the present disclosure. Like reference numerals in each drawing indicate like members.

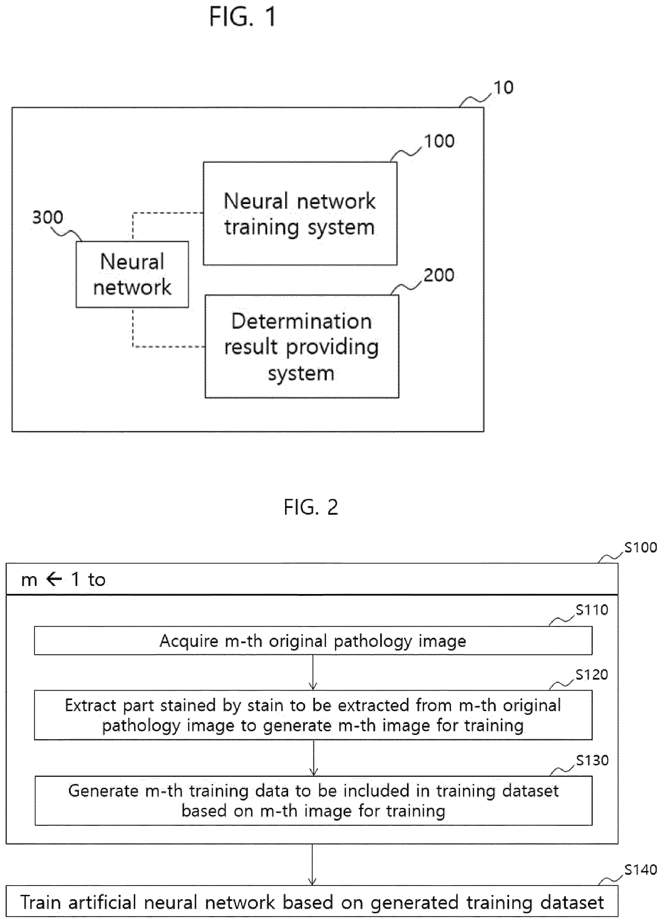

FIG. 1 is a schematic diagram illustrating an environment in which an artificial neural network training method and a determination result providing method for a pathology specimen are performed according to the technical idea of the present disclosure.

Referring to FIG. 1, the artificial neural network training method according to one embodiment of the present disclosure may be performed by a neural network training system 100, and the determination result providing method for a pathology specimen according to one embodiment of the present disclosure may be performed by a determination result providing system for a pathology specimen (200; hereinafter, referred to as a "determination result providing system"). The neural network training system 100 may train an artificial neural network 300 to detect lesions included in the pathology specimen, output diagnostic information for the pathology specimen, or provide prognostic information and/or response information to treatment methods, and the determination result providing system 200 may use the trained artificial neural network 300 to make various determinations about a target specimen (for example, determination on lesion detection, presence or absence of development of a disease, prognosis, treatment methods, etc.).

The neural network training system 100 and/or the determination result providing system 200 may be a computing system which is a data processing device having computing power to implement the technical idea of the present disclosure, and may generally include a computing device such as a personal computer or mobile terminal as well as a server which is a data processing device which is accessible by a client through a network.

The neural network training system 100 and/or the determination result providing system 200 may be implemented with any one physical device, but an average expert in the art to which the present disclosure pertains may easily deduce that the neural network training system 100 and/or the determination result providing system 200 can be implemented according to the technical idea of the present disclosure by organically combining a plurality of physical devices as needed.

The neural network training system 100 may train the neural network 300 based on training data generated from a large number of pathology specimens. The pathology specimens may be biopsies collected from various organs of the human body or biological tissues excised through surgery.

The neural network training system 100 may generate individual training data using a digital pathology image of the pathology specimen, and input it into an input layer of the neural network 300 to train the neural network 300.

The pathology image may be a digital slide image of the pathology specimen or a part of the digital slide image. A slide of the pathology specimen may be a sliced portion of the pathology specimen. The digital slide image of the pathology specimen may be generated by slicing the pathology specimen to produce a glass slide, staining the glass slide with a predetermined mixed stain, and digitizing it. In other words, the pathology image may be a pathology slide image obtained by producing a slide of the pathology specimen and staining it with a predetermined mixed stain (staining reagent), or an image obtained by dividing the stained pathology slide image into a predetermined size.

The pathology image may be a pathology image obtained by staining the slide of the pathology specimen with a mixed stain in which multiple individual stains (staining reagents) are mixed. The pathology image may be a whole slide image or a part of the whole slide image (for example, a patch image obtained by dividing the whole slide image into a predetermined size).

The mixed stain may be a stain that is a mixture of a predetermined stain to be extracted and one or more stains excluding the stain to be extracted.

In one embodiment, the stain to be extracted may be a hematoxylin stain (hereinafter, referred to as 'H-stain'), and hereinafter, the case where the stain to be extracted is the H-stain will be described as an example. However, the technical idea of the present disclosure is not limited thereto, and various types of stains may be determined as the stain to be extracted.

When the stain to be extracted is the H-stain, the mixed stain may be a hematoxylin-eosin stain (H&E stain), a mixed stain for the IHC staining method in which hematoxylin staining is a counterstain, or a mixed stain for a DAB staining method in which hematoxylin staining is a counterstain, and in addition, it may be various cocktail stains including the hematoxylin stain.

The neural network 300 may be an artificial neural network trained to output a probability value for whether a given disease is developed or whether a lesion is included. The neural network 300 may output a numerical value, i.e., a probability value, indicating a determination result for the target specimen (for example, the possibility of developing the disease) based on data input through the input layer.

In one embodiment, the artificial neural network 300 may be a patch-level classification neural network. The patch-level neural network may be a neural network which receives patch-level images as input and outputs values for classifying the patch.

In another embodiment, the artificial neural network 300 may be a pixel-level classification neural network. The pixel-level classification neural network may be a neural network which outputs a value for classifying each pixel included in an image.

In the present specification, the artificial neural network is a neural network artificially constructed based on operating principles of human neurons, and includes a multilayer perceptron model, and it may refer to a set of information expressing a series of design details which define an artificial neural network.

In one embodiment, the artificial neural network 300 may be a convolutional neural network or include a convolutional neural network.

On the other hand, the trained neural network 300 may be stored in the determination result providing system 200, and the determination result providing system 200 may make a determination about a predetermined diagnosis target specimen using the trained artificial neural network.

As shown in FIG. 1, the neural network training system 100 and/or the determination result providing system 200 may be implemented in the form of a subsystem of a predetermined parent system 10. The parent system 10 may be a server. The server 10 refers to a data processing device having computing power to implement the technical idea of the present disclosure, and In general, an average expert in the art to which the present disclosure pertains may easily deduce that not only data processing devices which is accessible by clients through a network, but also any device which can perform a specific service, such as a personal computer or mobile terminal, can be defined as the server.

Alternately, according to embodiments, the neural network training system 100 and the determination result providing system 200 may be implemented separately from each other.

FIG. 2 is a flowchart for illustrating a neural network training method according to one embodiment of the present disclosure.

Referring to FIG. 2, the neural network training system 100 may generate a training dataset including M pieces of individual training data (here, M is a natural number greater than or equal to 2). For this purpose, the neural network training system 100 may generate m-th training data to be included in the training dataset, for all m where 1<=m<=M (S100).

In order to generate the m-th training data to be included in the training dataset, the neural network training system 100 may acquire an m-th original pathology image (here, N is a natural number greater than or equal to 2) (S110).

According to embodiments, the neural network training system 100 may receive the m-th original pathology image corresponding to a predetermined pathology specimen from an external terminal, and may also obtain the m-th original pathology image corresponding to the pathology specimen from a memory device which stores the m-th original pathology image in advance.

The m-th pathology image may be a pathology image stained with a mixed stain in which a predetermined stain to be extracted (for example, hematoxylin stain) and one or more stains excluding the stain to be extracted are mixed.

The m-th pathology image may be labeled with predetermined information. The labeled information may vary depending on the purpose of the artificial neural network 300. For example, the information labeled in the m-th pathology image may be the presence or absence of a disease.

Alternatively, in the m-th original pathology image, a lesion area caused by a predetermined disease may be annotated. In other words, the m-th original pathology image may be tagged with annotation information representing the area of the lesion included in the m-th original pathology image.

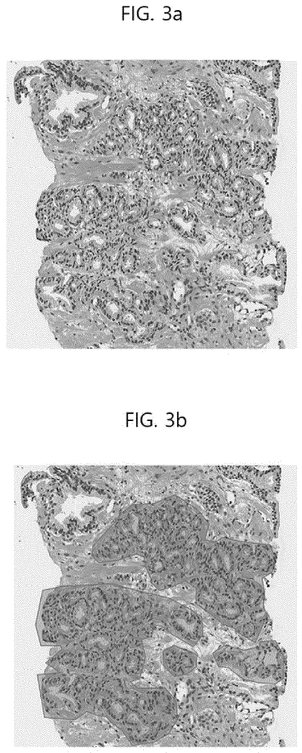

FIG. 3A is a diagram showing an example of the original pathology image, and FIG. 3B is a diagram showing a lesion area annotated to the pathology image of FIG. 3A. In the example of FIG. 3B, the area marked by the solid line represents the annotated lesion area.

Referring to FIG. 2 again, the neural network training system 100 may extract a part stained by the stain to be extracted (for example, hematoxylin stain) from the m-th original pathology image to generate an m-th pathology image for training (S120).

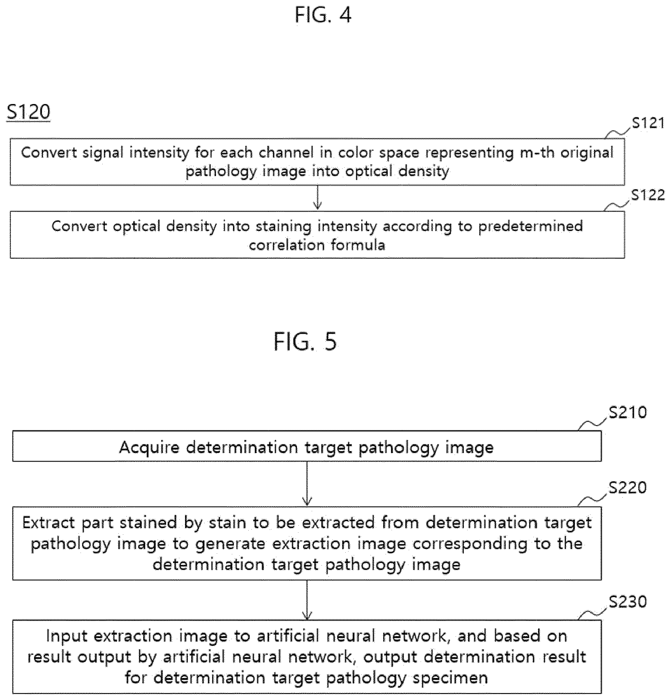

In one embodiment, the neural network training system 100 may perform color deconvolution to generate the m-th pathology image for training corresponding to the m-th original pathology image. FIG. 4 is an example illustrating a process of generating the m-th pathology image for training corresponding to the m-th original pathology image according to one embodiment of the present disclosure.

Referring to FIG. 4, the neural network training system 100 may convert a signal intensity for each channel in a color space representing the m-th original pathology image into optical density (S121). Thereafter, the neural network training system 100 can convert the optical density into staining intensity according to a predetermined correlation formula (S122).

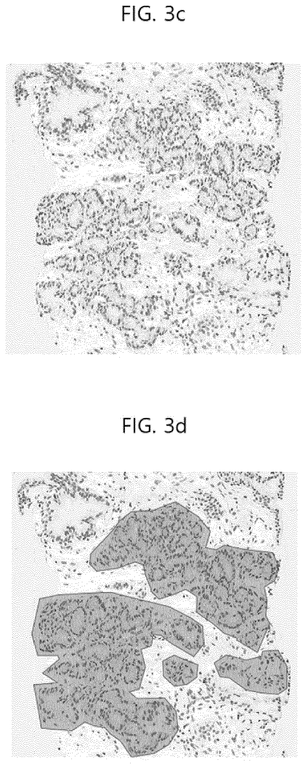

In addition, FIG. 3C is an example of an image extracted from the original pathology image shown in FIG. 3A of a part stained by the stain to be extracted (for example, hematoxylin stain).

Referring to FIG. 2 again, the neural network training system 100 may generate the m-th training data to be included in the training dataset based on the m-th pathology image for training (S130). More specifically, the neural network training system 100 may generate the m-th training data by tagging the m-th pathology image for training with the information labeled in the m-th original image.

For example, the m-th original image may have a lesion area annotated as shown in FIG. 3b. In this case, as shown in FIG. 3C, the generated m-th pathology image for training may be tagged with the annotation information of the m-th original image, an example of which is shown in FIG. 3D. In other words, FIG. 3D shows training data generated from the original pathology image tagged with the annotation information as shown in FIG. 3B.

Meanwhile, in the case that there is diagnostic information, prognostic information, and/or response information to a specific treatment method for the pathology specimen corresponding to the m-th training data, the neural network training system 100 may set the same as a label for the m-th training data.

When the training dataset including the M pieces of individual training data is generated through the above method, the neural network training system 100 may train the neural network 300 by inputting the generated training dataset into the input layer of the neural network 300 (S140 of FIG. 2). Since the deep learning technique of inputting training data tagged with correct answer information into the artificial neural network 300 and training the artificial neural network 300 through methods such as gradient descent and backpropagation is widely known, detailed description thereof will be omitted.

On the other hand, according to embodiments, M pathology images used to construct the training dataset may be stained with two or more different mixed stains. In other words, some of the first to M-th original pathology images and other parts of the first to M-th original pathology images may be pathology images stained with different mixed stains. Of course, two or more mixed stains used to stain the M pathology images may commonly include the stain to be extracted. For example, if M is 150, the first to 50th pathology images may be pathology images stained by the H&E staining method, and the 51st to 100th pathology images may be pathology images stained by the IHC staining method (counterstain is H-staining), and the 101st to 150th pathology images may be pathology images stained by the DAB staining method (counterstain is H-staining). Of course, according to embodiments, the M pathology images may be pathology images stained by four (4) or more different mixed stains.

As discussed above, the neural network training method according to the technical idea of the present disclosure extracts only the part stained by the stain to be extracted from the original pathology image and uses it as the training data of the artificial neural network. Therefore, through the neural network training method according to the technical idea of the present disclosure, there is an effect of capable of effectively performing training using pathology images produced by various staining methods using the stain to be extracted. In other words, the neural network training method according to the technical idea of the present disclosure has an effect of effectively training an artificial neural network that is able to make determination about a lesion or disease using images stained by different methods. Thus, even if multiple images used as the training data are not pathology images stained by the same method, there is an effect of capable of using them as training data that can train one artificial neural network (for example, 300) by the neural network training method.

FIG. 5 is a flowchart illustrating an example of a determination result providing method for a pathology specimen according to one embodiment of the present disclosure. The determination result providing method for a pathology specimen according to FIG. 5 may be performed by the determination result providing system 200, and the determination result providing system 200 may store the artificial neural network 300 trained by the neural network training system 100.

Referring to FIG. 5, the determination result providing system 200 may acquire a determination target pathology image of a predetermined determination target pathology specimen (S210). Here, the determination target pathology image may be a pathology slide image obtained by staining a slide of the determination target pathology specimen with a mixed stain in which the stain to be extracted (for example, hematoxylin stain) and one or more stains excluding the stain to be extracted (for example, hematoxylin stain) are mixed.

The determination result providing system 200 may extract the part stained by the stain to be extracted from the determination target pathology image and generate an extraction image corresponding to the determination target pathology image (S220). Since a process of generating input data corresponding to the determination target pathology image of the determination target specimen is very similar to the process as described above, separate explanation will be omitted.

The determination result providing system 200 may input the input data to the artificial neural network 300, and based on a result output by the artificial neural network, output a determination result for the determination target pathology specimen (S230).

On the other hand, the staining method used to stain at least a part of the original pathology images used to generate the training data and the staining method used to stain the determination target pathology image may be different. In other words, at least a part of the determination target image and a part of the images used to train the artificial neural network 300 (the first to M-th original pathology images) may be pathology images stained with different mixed stains. For example, all or at least a part of the images used to train the artificial neural network 300 may be pathology images stained by the H&E staining method, but the determination target image may be an image stained by the IHC method.

Alternatively, the staining method used to stain the determination target image may be different from a method of staining any of the images used to train the artificial neural network 300. For example, the staining method used to stain the determination target image is the DAB method, but all images used to train the artificial neural network 300 may be images stained using the H&E staining method.

In the case of conventional determination technology for pathology images using artificial neural networks, training and determination are made on pathology images stained by a specific method, but according to the technical idea of the present disclosure, there is an effect of enabling training and determination through images stained with different staining methods. For example, there is an effect of capable of detecting cancerous lesions by analyzing pathology images generated from slides produced by staining a specific target with DAB or other colors and staining cell nuclei with hematoxylin, regardless of the type of target.

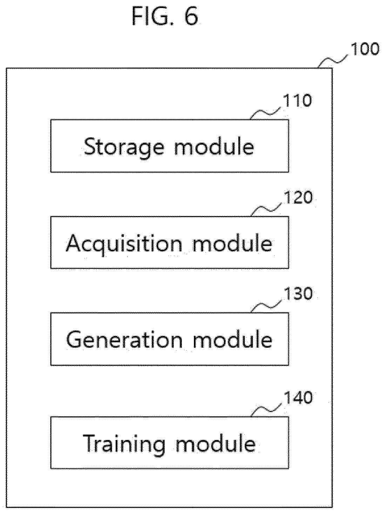

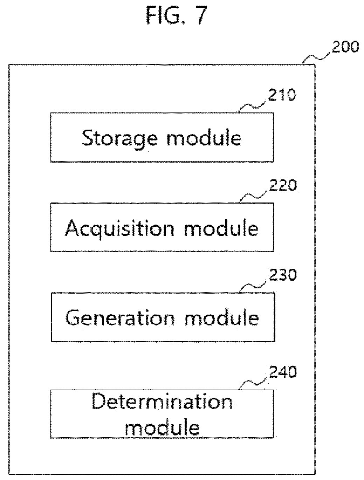

FIG. 6 is a diagram illustrating a schematic configuration of the artificial neural network training system 100 according to one embodiment of the present disclosure, and FIG. 7 is a diagram illustrating a schematic configuration of the determination result providing system 200 according to one embodiment of the present disclosure.

The artificial neural network training system 100 and the determination result providing system 200 may mean a logical configuration with hardware resources and/or software required to implement the technical idea of the present disclosure, and does not necessarily mean a single physical component or a single device. In other words, the artificial neural network training system 100 and the determination result providing system 200 may mean a logical combination of hardware and/or software provided to implement the technical idea of the present disclosure and, if necessary, may be implemented as a set of logical configurations to implement the technical idea of the present disclosure by being installed on devices separated from each other and performing their respective functions. In addition, the artificial neural network training system 100 and the determination result providing system 200 may mean a set of configurations that are implemented separately for each function or role to implement the technical idea of the present disclosure. Each configuration of the artificial neural network training system 100 and the determination result providing system 200 may be located on different physical devices or on the same physical device. Further, according to implementation examples, combinations of software and/or hardware that constitute respective components of the artificial neural network training system 100 and the determination result providing system 200 are also located in different physical devices, and the configurations located in the different physical devices may be organically combined with each other to implement each module.

In addition, the term "module" in this specification may mean a functional and structural combination of hardware for carrying out the technical idea of the present disclosure and software for driving the hardware. For example, an average expert in the art to which the present disclosure pertains may easily deduce that the module may mean a logical unit of a predetermined code and hardware resources for executing the predetermined code and does not necessarily mean a physically connected code or a single type of hardware.

Referring to FIG. 6, the artificial neural network training system 100 may include a storage module 110, an acquisition module 120, a generation module 130, and a training module 140. According to embodiments of the present disclosure, some of the above-described components may not necessarily correspond to the components essential for the implementation of the present disclosure, and of course, the artificial neural network training system 100 may include more components than these. For example, the artificial neural network training system 100 may further include a communication module (not shown) for communicating with an external device, and a control module (not shown) for controlling the components and resources of the artificial neural network training system 100.

The storage module 110 may store an artificial neural network 40 to be trained.

The acquisition module 120 may acquire M original pathology images stained with a mixed stain in which the predetermined stain to be extracted and one or more stains excluding the stain to be extracted are mixed.

The generation module 130 may generate individual training data based on each original pathology image, and may construct the training dataset including a plurality pieces of individual training data.

The training module 140 may train the artificial neural network 300 based on the training dataset.

Referring to FIG. 7, the determination result providing system 200 may include a storage module 210, an acquisition module 220, a generation module 230, and a determination module 240. According to embodiments of the present disclosure, some of the above-described components may not necessarily correspond to the components essential to implementation of the present disclosure, and of course, the determination result providing system 200 may include more components according to embodiments. For example, the determination result providing system 200 may further include a communication module (not shown) to communicate with a three-axis vibration sensor 20, and a control module (not shown) to control the components and resources of the determination result providing system 200.

The storage module 210 may store the trained artificial neural network 40.

The acquisition module 220 may acquire the determination target pathology image obtained by staining the predetermined determination target pathology specimen with the mixed stain in which one or more stains excluding the stain to be extracted are mixed.

The generation module 230 may generate the input data based on the determination target pathology image.

The determination module 240 may input the input data to the artificial neural network 300, and make determination on the determination target specimen based on a prediction value output from the artificial neural network 300.

On the other hand, according to implementation examples, the artificial neural network training system 100 and the determination result providing system 200 may include a processor and a memory to store a program executed by the processor. The processor may include a single-core CPU or a multi-core CPU. The memory may include high-speed random access memory and may include non-volatile memory, such as one or more magnetic disk storage devices, flash memory devices, or other non-volatile solid-state memory devices. Access to memory by the processor and other components may be controlled by a memory controller.

In addition, the method according to the embodiment of the present disclosure may be implemented in the form of computer-readable program instructions and stored in a non-transitory computer-readable recording medium, and a control program and its target program according to an embodiment of the present disclosure may also be stored in a non-transitory computer-readable recording medium. A non-transitory computer-readable recording medium includes any type of recording device in which data that can be read by a computer system is stored.

The program instructions recorded on the recording medium may be specially designed and configured for the present disclosure or may be known and available to a person skilled in the field of software.

Examples of non-transitory computer-readable recording media include magnetic media such as hard disks, floppy disks, and magnetic tapes, optical media such as CD-ROMs and DVDs, magneto-optical media such as floptical disks, and hardware devices specially configured to store and execute program instructions, such as ROMs, RAMs, flash memory, and the like. In addition, non-transitory computer-readable recording media may be distributed across networked computer systems, where computer-readable codes may be stored and executed in a distributed manner.

Examples of program instructions include machine language code, such as those generated by a compiler, as well as high-level language code that can be executed by a device that processes information electronically using an interpreter such as a computer.

The hardware devices described above may be configured to operate as one or more software modules to perform the operations of the present disclosure, and vice versa.

The foregoing description of the present disclosure is for illustrative purposes, and those skilled in the art will understand that the present disclosure may be easily modified into other specific forms without changing the technical idea or essential features of the present disclosure. Therefore, the embodiments described above should be understood in all respects as illustrative and not restrictive. For example, each component described as unitary may be implemented in a distributed manner, and similarly, components described as distributed may also be implemented in a combined form.

The scope of the present disclosure is indicated by the appended claims rather than the detailed description above, and all changes or modified forms derived from the meaning and scope of the claims and their equivalent concepts should be construed as being included in the scope of the present disclosure.

### Industrial Applicability

The present disclosure may be used for a training method of an artificial neural network which provides a determination result for a pathology specimen, and a computing system which performs the same.

## Claims

1. A method of training an artificial neural network, the method comprising:
generating, by a neural network training system, a training dataset including M pieces of individual training data (where M is a natural number greater than or equal to 2); and
training, by the neural network training system, the artificial neural network based on the training dataset,
wherein the generating of the training dataset including the M pieces of individual training data comprises generating m-th training data to be included in the training dataset for all m where 1 <=m<=M, and
the generating of the m-th training data comprise:
acquiring an m-th original pathology image, where the m-th original pathology image is a pathology image stained with a mixed stain in which a predetermined stain to be extracted and one or more stains excluding the stain to be extracted are mixed;
extracting a part stained by the stain to be extracted from the m-th original pathology image to generate an m-th pathology image for training corresponding to the m-th original pathology image; and
generating the m-th training data based on the m-th pathology image for training.

2. The method of claim 1, wherein the stain to be extracted is a hematoxylin stain.

3. The method of claim 1, wherein a part of the first to M-th original pathology images and another part of the first to M-th original pathology images are pathology images stained with different mixed stains.

4. The method of claim 1, wherein the extracting of the part stained by the stain to be extracted from the m-th original pathology image to generate the m-th pathology image for training corresponding to the m-th original pathology image comprises:
converting a signal intensity for each channel in a color space representing the m-th original pathology image into optical density; and
converting the optical density into staining intensity according to a predetermined correlation formula.

5. The method of claim 1, wherein a lesion area caused by a predetermined disease is annotated in the m-th original pathology image, and
the generating of the m-th training data based on the m-th pathology image for training comprises generating the m-th training data by annotating the lesion area annotated in the m-th original pathology image to the m-th pathology image for training.

6. A method of providing a determination result for a predetermined determination target pathology specimen through an artificial neural network trained by the method according to claim 1, the method comprising:
acquiring, by a computing system, a determination target pathology image stained with a mixed stain in which the stain to be extracted and one or more stains excluding the stain to be extracted are mixed;
generating, by the computing system, an extraction image corresponding to the the determination target pathology image by extracting a part stained by the stain to be extracted from the determination target pathology image; and
outputting, by the computing system, a determination result for the determination target pathology specimen by the artificial neural network based on the extraction image.

7. The method of claim 6, wherein at least a part of the determination target pathology image and a part of the first to M-th original pathology images are pathology images stained with different mixed stains.

8. A computer program installed in a data processing device and recorded on a non-transitory medium for performing the method according to any one of claims 1 to 7.

9. A non-transitory computer-readable recording medium on which a computer program for performing the method according to any one of claims 1 to 7 is recorded.

10. An artificial neural network training system comprising:
a processor; and
a memory in which a computer program is stored,
wherein the computer program is configured to, when executed by the processor, cause the artificial neural network training system to perform a method of training an artificial neural network, and
the method of training the artificial neural network comprises:
generating, by the neural network training system, a training dataset including M pieces of individual training data (where M is a natural number greater than or equal to 2); and
training, by the neural network training system, the artificial neural network based on the training dataset,
wherein the generating of the training dataset including the M pieces of individual training data comprises generating m-th training data to be included in the training dataset for all m where 1<=m<=M, and
the generating of the m-th training data comprise:
acquiring an m-th original pathology image, where the m-th original pathology image is a pathology image stained with a mixed stain in which a predetermined stain to be extracted and one or more stains excluding the stain to be extracted are mixed;
extracting a part stained by the stain to be extracted from the m-th original pathology image to generate an m-th pathology image for training corresponding to the m-th original pathology image; and
generating the m-th training data based on the m-th pathology image for training.

11. The artificial neural network training system of claim 10, wherein the stain to be extracted is a hematoxylin stain.

12. The artificial neural network training system of claim 10, wherein a part of the first to M-th original pathology images and another part of the first to M-th original pathology images are pathology images stained with different mixed stains.

13. The artificial neural network training system of claim 10, wherein a lesion area caused by a predetermined disease is annotated in the m-th original pathology image, and
the generating of the m-th training data based on the m-th original pathology image comprises generating the m-th training data by annotating the lesion area annotated in the m-th original pathology image to the m-th pathology image for training.

14. The artificial neural network training system of claim 10, wherein the extracting of the part stained by the stain to be extracted from the m-th original pathology image to generate the m-th pathology image for training corresponding to the m-th original pathology image comprises:
converting a signal intensity for each channel in a color space representing the m-th original pathology image into optical density; and
converting the optical density into staining intensity according to a predetermined correlation formula.

15. A determination result providing system for a pathology specimen, comprising:
a processor; and
a memory in which a computer program is stored,
wherein the computer program is configured to, when executed by the processor, cause the determination result providing system to perform a method of providing a determination result for the pathology specimen through an artificial neural network trained by the method according to claim 1, and
the method of providing the determination result comprises:
acquiring, by the determination result providing system, a determination target pathology image stained with a mixed stain in which the stain to be extracted and one or more stains excluding the stain to be extracted are mixed;
generating, by the determination result providing system, an extraction image corresponding to the the determination target pathology image by extracting a part stained by the stain to be extracted from the determination target pathology image; and
outputting, by the determination result providing system, a determination result for the determination target pathology specimen by the artificial neural network based on the extraction image.

16. The determination result providing system of claim 15, wherein at least a part of the determination target image and a part of the first to M-th original pathology images are pathology images stained with different mixed stains.
